Europäisches Patentamt

European Patent Office

Office européen des brevets

(19)

(11) EP 1 659 406 A1

(12) DEMANDE DE BREVET EUROPEEN

(43) Date de publication:
24.05.2006 Bulletin 2006/21

(51) Int Cl.:
*G01N 33/74* (2006.01)    *G01N 33/53* (2006.01)
*C07K 16/26* (2006.01)

(21) Numéro de dépôt: 04447260.3

(22) Date de dépôt: 23.11.2004

(84) Etats contractants désignés:
AT BE BG CH CY CZ DE DK EE ES FI FR GB GR
HU IE IS IT LI LU MC NL PL PT RO SE SI SK TR
Etats d'extension désignés:
AL HR LT LV MK YU

(71) Demandeur: Biocode-Hycel
4000 Liege (BE)

(72) Inventeurs:
• Pirens, Guy
4020 Liege (BE)

• Hennen, George
4920 Aywaille (BE)
• Sarlet, Guy
4550 Nandrin (BE)

(74) Mandataire: Van Malderen, Joëlle et al
pronovem - Office Van Malderen
Avenue Josse Goffin 158
1082 Bruxelles (BE)

(54) **Procédé de détection et de dosage direct de macroprolactines et/ou de prolactines**

(57)    La présente invention est relative à un procédé de détection et/ou de dosage de macroprolactines et de prolactine monomérique combinant deux immunodosages qui permettent la détermination directe d'une hyperprolactinémie et la détection et la semi-quantification de macroprolactines présentes dans un même échantillon biologique extrait d'un patient humain ou animal.

L'objet de l'invention concerne également les éléments pour la mise en oeuvre de ce procédé.

Figure 6

EP 1 659 406 A1

## Description

### Objet de l'invention

La présente invention est relative à un procédé de détection et/ou de dosage (direct) de macroprolactines et/ou de prolactine monomérique, à des fins de diagnostic humain ou vétérinaire, en particulier pour le suivi de différentes pathologies influencées par des concentrations élevées en prolactines et éventuellement en macroprolactine.

La présente invention concerne également les moyens pour la mise en oeuvre de ce procédé, en particulier une trousse de diagnostic ou un dispositif incorporant lesdits moyens destinés à la mise en oeuvre de ce procédé, en particulier un automate permettant d'effectuer automatiquement les étapes de cette détection et/ou ce dosage.

### Arrière-plan technologique et état de la technique à la base de l'invention

La prolactine (PRL) est une hormone, dont une faible proportion est N-glycosylée, composée de cent quatre-vingt dix neuf acides aminés. Cette hormone est sécrétée par les cellules lactotropes de l'hypophyse antérieure. Cette sécrétion, activement soutenue par la lactation, est physiologiquement réprimée par la dopamine hypothalamique et être activée par différents stress, y compris la stimulation du mamelon lors de la tétée.

Le rôle physiologique principal de cette hormone est le développement de la glande mammaire en préalable à la lactation et l'entretien de la lactation dans le post-partum. En outre, cette hormone a également d'autres implications au niveau d'autres organes, en particulier au niveau des fonctions gonadiques et de la prostate.

Une concentration élevée en prolactine (hyperprolactinémie) est un marqueur de pathologies éventuellement graves. L'hyperprolactinémie entraîne des situations pathologiques où dominent des anomalies des fonctions gonadiques avec ou sans galactorrhée.

Cette pathologie peut avoir pour cause principale un prolactinome qui est l'adénome hypophysaire le plus fréquent et qui provoque une sécrétion de la prolactine en excès, parfois accompagnée d'autres hormones, telles que la GH (hormone de croissance) ou la TSH (hormone thyréostimulante). Il doit être suspecté dans toutes les situations cliniques pouvant dépendre d'une hyperprolactinémie.

L'hyperprolactinémie (PRL) peut être détectée par une recherche éthiopathogénique dans de très nombreuses situations affectant un homme ou une femme et ce plus particulièrement dans les cas suivants : irrégularité du cycle menstruel et/ou de l'ovulation, aménorrhée, galactorrhée, hypogonadisme féminin infertilité masculine, troubles érectiles et de la libido, gynécomastie, syndrome de masse pituitaire, lésion du système nerveux central, etc.

Cependant, il est important de noter qu'une majorité de patients peut présenter une prolactinémie apparente élevée sans induire nécessairement une pathologie sévère, tel qu'un développement tumoral hypophysaire.

Néanmoins, la mise en évidence d'une hyperprolactinémie oriente la conduite médicale de façon déterminante dans le sens d'une recherche d'un prolactinome, d'un adénome hypophysaire mixte à GH-PRL, à TSH-PRL, d'un macroadénome non secrétant, d'un gonadotropinome, d'une hypophysite auto-immune et de toutes les causes secondaires d'hyperprolactinémie pathologique telles qu'une hypothyroïdie, une cirrhose hépatique, une insuffisance rénale et des causes pharmacologiques freinant la production (ou les effets) de la dopamine hypothalamique.

Cependant, les situations cliniques s'exprimant par des déficiences gonadiques ou sexuelles sont loin d'être systématiquement dues à une hyperprolactinémie.

Par conséquent, il existe un besoin de détecter de manière spécifique une hyperprolactinémie liée spécifiquement aux situations pathologiques significatives.

Cependant, plus de 20% des patients classés hyperprolactinémiques par les procédés habituels, c'est-à-dire par des dosages de routine, présentent diverses formes de prolactines circulantes impliquées partiellement dans différents complexes. On peut diviser ces complexes en deux groupes:

- des dimères de prolactine (Big-prolactine) présents de manière peu fréquente et par conséquent interférant peu dans le dosage de la prolactine ;
- des complexes de prolactine (Big-Big-prolactine ou macroprolactine) de poids moléculaires (PM) supérieurs à 150 kD (PM élevés comparativement au PM de la prolactine monomérique: environ 20 kDa) et composés essentiellement par association de prolactine monomérique à des auto-anticorps.

Cette dernière catégorie interfère dans les dosages de la prolactine monomérique.

De plus, ces complexes ne sont que rarement suffisamment actifs sur le plan hormonal pour générer un tableau clinique caractéristique.

Habituellement, un dosage correct de la prolactine dans un sérum est rendu possible par isolement de celle-ci via des techniques de séparation complexe (gel-filtration, précipitation par le polyéthylène glycol (PEG) ou la protéine A-Sépharose) qui présentent l'inconvénient de la complexité de leur mise en oeuvre. Une détermination exacte du contenu en prolactine monomérique dans un sérum dans un sérum nécessite par conséquent un traitement préalable du sérum avant son dosage immunologique proprement dit.

Outre leur coût élevé et leur complexité, ces techniques présentent généralement un défaut de sensibilité lié à la méthode de précipitation ou à la dilution des échantillons dans le cas du procédé chromatographique.

## Buts de l'invention

La présente invention vise à fournir un procédé et des moyens (trousse ou appareil) de détection et/ou de dosage des macroprolactines et/ou de la prolactine (monomérique) dans un échantillon biologique extrait d'un patient pour améliorer le diagnostic et éventuellement le suivi des différentes pathologies liées à une hyperprolactinémie ; ce procédé et ces moyens ne présentant pas les inconvénients de l'état de la technique.

La présente invention vise en particulier à fournir un tel procédé et de tels moyens de détection et/ou de dosage qui ne nécessitent pas un traitement préalable de l'échantillon biologique testé.

Un autre but de la présente invention est de fournir un tel procédé et de tels moyens permettant une automatisation des étapes de détection et/ou de dosage.

Un dernier but de la présente invention est de fournir un procédé et des moyens de détection et/ou de dosage qui présentent une sensibilité et une spécificité suffisamment grandes pour éviter les faux positifs et les faux négatifs au niveau de la détection et/ou du dosage d'une hyperprolactinémie et éventuellement d'une hypermacroprolactinémie.

## Eléments caractéristiques de la présente invention

La présente invention est relative à un procédé de détection et/ou de dosage de macroprolactines et de prolactine (monomérique) combinant deux immunodosages qui permettent la détermination directe d'une hyperprolactinémie et la détection et la semi-quantification de macroprolactines présentes dans un même échantillon biologique extrait d'un patient (humain ou animal). Ce procédé comprend différentes étapes (dans lequel les étapes suivantes a et b peuvent être éventuellement inversées):

a. un immuno-dosage, de préférence de type IRMA/ELISA/ILMA ainsi qu'un dosage de type compétitif par la capture préférentielle de la prolactine sous une forme monomérique présente dans l'échantillon biologique à doser par l'utilisation d'anticorps ou des portions hypervariables d'anticorps reconnaissant spécifiquement ladite prolactine monomérique (tout en reconnaissant une présence de cette dernière, la moins interférée possible avec la présence éventuelle de macroprolactines dans l'échantillon biologique) et évaluation d'un premier titre (titre 1) en prolactine dans l'échantillon biologique par l'interpolation du signal obtenu dans le dosage de l'échantillon biologique avec ceux obtenus dans le dosage d'une courbe standard) (c'est-à-dire une courbe de dilution calibrée sur le standard international WHO84/500 composée uniquement de prolactine monomérique) ;

b. un immunodosage, de préférence (de type IRMA/ELISA/ILMA) ainsi qu'un dosage de type compétitif par la capture préférentielle de la prolactine sous forme de macroprolactines, éventuellement présentes dans l'échantillon biologique à doser par des anticorps ou des portions hypervariables d'anticorps reconnaissant préférentiellement ladite prolactine sous forme de macroprolactines (tout en présentant éventuellement une reconnaissance de ces dernières la moins interférée possible par la prolactine monomérique présente dans l'échantillon biologique) et évaluation d'un second titre (titre 2) en prolactine dans l'échantillon biologique par interpolation du signal obtenu dans le dosage d'échantillon biologique avec ceux obtenus lors du dosage d'une courbe standard, de préférence similaire à celle utilisée dans l'étape a) (c'est-à-dire une courbe de dilution calibrée sous le standard international WHO84/500 composée uniquement de prolactine monomérique) et éventuellement;

c. un établissement d'une corrélation entre ces deux immunodosages obtenus à la présence ou l'absence d'une hyperprolactinémie chez le patient dans le cas où :

- si le titre 1 est inférieur à la limite supérieure de l'intervalle de normalité d'un patient non affecté par une hyperprolactinémie, on considère, sous réserve de surveillance ultérieure, l'échantillon biologique comme étant obtenu d'un patient sain (non soumis à une hyperprolactinémie);
- si le titre 1 est supérieur à la limite supérieure de l'intervalle de normalité, on considère que l'échantillon biologique est obtenu à partir d'un patient souffrant d'une hyperprolactinémie (dite vraie) et/ou éventuellement d'une macroprolactinémie ;
- si le titre 2 est comparable au titre 1, on considère que l'échantillon biologique est obtenu à partir d'un patient souffrant uniquement d'une hyperprolactinémie (dite vraie) et,
- si le titre 2 est significativement supérieur au titre 1, on considère que l'échantillon comporte des macroprolactines et est obtenu à partir d'un patient présentant une macroprolactinémie d'autant plus élevée que le différentiel entre le titre 2 et le titre 1 est important ;

- si les titres 1 et 2 sont très supérieurs aux limites supérieures des intervalles de normalité et que la différence entre le titre 2 et le titre 1 révèle la présence d'une macroprolactinémie incompatible avec une prolactinémie identifiée par le titre 1, on considère que l'échantillon biologique révèle la présence chez le patient d'une hyperprolactinémie (dite vraie), éventuellement associée à la présence d'une macroprolactinémie.

Dans le procédé de l'invention, les immunodosages sont obtenus par des techniques bien connues de l'homme de l'art, notamment des immuno-dosages de type IRMA, ELISA ou ILMA, de préférence des dosages sandwichs en une ou deux étapes nécessitant éventuellement une opération de lavage pour éliminer les contaminants intervenant dans la fixation de la prolactine et/ou des macroprolactines sur les anticorps ou des portions hypervariables de ces anticorps. Ces différents procédés d'immunodosages comportent de préférence une fixation des anticorps ou des portions hypervariables d'anticorps sur un support solide adéquat avant la mise en contact de l'échantillon biologique avec les anticorps ou les portions hypervariables de ceux-ci sur le support solide.

Cet immunodosage comporte également une révélation de la fixation des macroprolactines ou de la prolactine (monomérique) sur les anticorps ou des portions hypervariables de ceux-ci par l'utilisation d'un second marqueur, de préférence, un anticorps ou une portion hypervariable d'un anticorps réagissant avec un autre épitope de la prolactine ou des macroprolactines, ledit marqueur, de préférence ledit anticorps ou ladite portion hypervariable de l'anticorps comportant également un réactif susceptible d'être détecté par un signal.

Ce réactif peut être un réactif radioactif, une enzyme, un élément fluorescent, etc, tel que proposé dans les procédés d'immunodosage bien connu de l'homme de l'art. De même, les caractérisations des titres s'effectuent par des méthodes de dosage bien connues de l'homme de l'art dans le domaine immunodiagnostic.

Dans le procédé de l'invention, on entend par anticorps ou des portions hypervariables d'anticorps reconnaissant spécifiquement une prolactine monomérique ou une macroprolactine, des anticorps de préférence monoclonaux ou des portions hypervariables d'anticorps monoclonaux dirigés spécifiquement contre ces molécules et présentant une forte immunoaffinité pour ces molécules. Ce type d'anticorps ou ces portions hypervariables d'anticorps peuvent éventuellement présenter également une faible affinité pour d'autres molécules, cette faible immunoaffinité n'interférant pas avec l'immunodosage spécifique de chacune de ces molécules.

Dans le procédé et la trousse de diagnostic de l'invention, l'anticorps reconnaissant préférentiellement la macroprolactine est identifié dans le descriptif de l'invention comme étant l'anticorps 68.9.G produit par un hybridome portant la référence LMBP 6348CB. Ce dépôt LMBP 6348CB a été effectué selon le traité de Budapest le 9 novembre 2004 auprès de la collection BCCM/LMBP - Universiteit Gent, Technologiepark 927 (FSVM-bldg), B-9052 Gent-Zwijnaarde, BELGIUM.

Dans le procédé de détection et/ou de dosage de l'invention, l'échantillon biologique (humain ou animal) est de préférence choisi parmi le groupe constitué par le sang veineux ou capillaire, le sérum ou le plasma.

La phase solide sur laquelle sont fixés les anticorps dans l'immunodosage est constituée par tout type de support solide adéquat, de préférence des supports solides permettant une automatisation du procédé de détection et/ou de dosage, par exemple des plaques de multipuits ou des billes ou particules de taille adéquate, éventuellement magnétiques ou tout autre type de support, en particulier des tubes en plastique.

Un autre aspect de la présente invention concerne l'anticorps monoclonal utilisé dans l'immunodosage de l'invention et reconnaissant préférentiellement les macroprolactines ou une portion hypervariable de cet anticorps, de préférence cet anticorps est un anticorps monoclonal correspondant à l'anticorps 68.9G5 produit par l'hybridome LMBP 6348CB.

Un autre aspect de la présente invention concerne le support solide comportant, fixé directement ou indirectement (par exemple via deux molécules complémentaires, telles que de la biotine et de l'avidine ou de la biotine et de la streptavidine) à sa surface, lesdits anticorps ou les portions hypervariables desdits anticorps reconnaissant préférentiellement les macroprolactines et éventuellement sur une autre partie de la surface du support solide des anticorps ou des portions hypervariables desdits anticorps reconnaissant spécifiquement la prolactine monomérique.

L'objet de l'invention concerne également ce couple de deux types différents d'anticorps reconnaissant respectivement et préférentiellement les macroprolactines et la prolactine monomérique.

Ces couples de deux types d'anticorps peuvent être présents dans une trousse (kit of parts), en particulier une trousse de diagnostic et/ou d'immunodosage ou un appareillage (en particulier un automate) de diagnostic et/ou d'immunodosage pour doser la prolactine et/ou détecter éventuellement simultanément les macroprolactines dans un même échantillon biologique extrait d'un patient.

Cette trousse ou cet appareillage comportant également les autres éléments nécessaires pour effectuer le procédé de détection et/ou de dosage de l'invention, en particulier des solutions standards comprenant des dilutions variables en prolactine monomérique et/ou en macroprolactines dans une matrice adaptée aux échantillons à doser et éventuellement des blancs (contrôles) permettant de vérifier l'efficacité de l'immunodosage de l'invention.

Un dernier aspect de la présente invention concerne l'hybridome produisant des anticorps reconnaissant préférentiellement les macroprolactines, de préférence cet hybridome correspond à l'hybridome déposé sous la référence LMBP 6348CB.

Selon une variante de l'exécution du procédé de l'invention, il est également possible de proposer uniquement un immunodosage des macroprolactines présentes dans un échantillon biologique extrait d'un patient (humain ou animal) par des anticorps ou des portions hypervariables de ces anticorps reconnaissant préférentiellement les macroprolactines et l'évaluation d'un titre en macroprolactines dans l'échantillon biologique par interpolation du signal obtenu dans le dosage de l'échantillon biologique avec ceux obtenus lors du dosage d'une courbe standard (éventuellement calibré avec différentes dilutions selon un standard composé de macroprolactines ou d'un mélange de macroprolactines et de prolactine monomérique ou uniquement un standard international composé de prolactine monomérique). Cet immuno-dosage peut simplement servir pour la détection directe de ces macroprolactines sans effectuer éventuellement de dosage desdites macroprolactines et/ou du dosage de la prolactine monomérique. Cette détection et/ou ce dosage d'hyper(macro)prolactinémie pouvant servir au suivi de cet état en tant que tel puisqu'il est reconnu par quelque 5% des cas de macroprolactinémie accompagnée à un moment de leur évolution de lésions hypothalamo-hypophisaires par exemple, ou d'autres pathologies importantes pour le suivi en clinique de patients, telles que des maladies auto-immunes, par exemple.

Selon un dernier aspect de la présente invention, cette forme d'exécution préférée du procédé de l'invention peut être combinée à un procédé de discrimination des macroprolactines par rapport à la prolactine monomérique dans un échantillon biologique, de préférence un sérum, qui comprendrait les étapes suivantes :

- on détermine une première concentration en prolactine $[PRL]_I$ selon un immunodosage commercial comparable aux conditions d'un immunodosage, tel que décrit précédemment, à savoir dans lequel la capture de la prolactine monomérique s'effectue préférentiellement à celle des macroprolactines ;
- on détermine une deuxième concentration en prolactine $[PRL]_{II}$ selon le procédé de l'invention (de préférence réalisée en deux étapes) dans lequel la capture de la prolactine monomérique et/ou des macroprolactines effectuée à l'aide de l'anticorps ou d'une portion hypervariable de l'anticorps de l'invention, c'est-à-dire l'anticorps monoclonal produit par l'hybridome portant le numéro de dépôt LMBP 6348CB et dans lequel la révélation est réalisée à l'aide d'un anticorps signal, de préférence un anticorps marqué, (par exemple l'anticorps 69.2F11 utilisé dans l'exemple de l'invention) et on utilise pour l'interprétation du résultat, un algorithme adapté à titre d'exemple, le rapport R = $[PRL]_{II}$ - $[PRL]_I$ / $[PRL]_{II}$ qui permet ensuite d'établir sur base expérimentale un seuil de positivité du facteur R permettant d'affirmer que l'échantillon biologique contient des macroprolactines et si R = 0, c'est que l'échantillon biologique ne contient que de la prolactine sous forme monomérique.

## Description détaillée de l'invention

### *Dosage de la prolactine*

La détermination de la prolactine dans un échantillon biologique extrait d'un patient humain ou animal (en particulier le sang) se fait généralement sur un échantillon de sérum ou de plasma par une méthode immunométrique. Dans ce procédé de dosage, la substance de référence est le standard international dénommé PRL WHO 84/500 qui est constitué exclusivement de prolactine monomérique hypophysaire purifiée.

Selon les propriétés des anticorps poly- ou monoclonaux utilisés dans ces méthodes, les macroprolactines s'expriment selon une activité immunologique qui ne peut être quantifiée par manque de standard adéquat. Les résultats sont dès lors exprimés en terme de prolactine monomérique WHO 84/500.

Dès que le résultat dépasse la limite supérieure de l'intervalle normal de mesure, l'hyperprolactinémie doit être caractérisée en une hyperprolactinémie vraie ou en une macroprolactinémie.

Le procédé de l'invention comprend l'utilisation d'anticorps monoclonaux qui, utilisés en couples adéquats dans des techniques immunométriques, permettent une détermination directe sur un même échantillon de sérum de la prolactine monomérique tout en révélant la présence de macroprolactines et ce sans utiliser un traitement quelconque de l'échantillon.

Le procédé et la trousse de l'invention reposent sur l'utilisation de deux couples d'anticorps monoclonaux présentant des caractéristiques fonctionnelles opposées. Le premier couple est composé d'une paire d'anticorps reconnaissant préférentiellement la prolactine sous une forme monomérique. Les anticorps de la seconde paire sont au contraire choisis pour leur capacité de fixation de la prolactine sous toutes ces formes et en particulier sous forme de macroprolactine (c'est-à-dire au moins un des deux anticorps doit présenter une plus forte affinité pour les macroprolactines que pour la prolactine monomérique). L'invention permettant la détection et/ou le dosage de la prolactine monomérique et la détection et/ou le dosage des macroprolactines repose sur l'interprétation des résultats obtenus lors de 2 détections et dosages réalisés avec les 2 couples d'anticorps sélectionnés. Ces dosages sont de préférence calibrés sur base d'un standard adéquat, en particulier du standard international WHO 84/500 composé de prolactine monomérique.

Des études cliniques ont attesté de l'excellente corrélation entre les résultats obtenus sur base de l'invention et ceux obtenus par la méthode de précipitation au polyéthylène glycol (PEG) et par une méthode chromatographique.

De plus, les patients étudiés ont été explorés complètement sur le plan endocrinien et l'imagerie hypothalamo-hypophysaire par des endocrinologues qui ont confirmé par une analyse clinique les détections positives effectuées par le procédé de l'invention.

D'autres caractéristiques du procédé et des moyens de dosage de l'invention seront décrites en détails, en référence aux figures identifiées ci-dessous dans les exemples ci-dessous présentés à titre d'illustration non limitative de l'invention.

## Brève description des figures

La figure 1 représente la différence de réactivité immunologique de la PRL monomérique dans deux systèmes de dosage différents.

La figure 2 représente le profil d'élution d'un set de calibrateurs protéiques (Cali. Pharmacia LMW ref 17044601) et de quelques protéines supplémentaires de référence (Thyroglobuline (Tg), ferritine et une IgG) sur une colonne de chromatographie superdex 200 (HR 10/30 de pharmacia). Ce profil a été utilisé pour calibrer une colonne et déterminer ces performances.

La figure 3 illustre les performances (Kav vs Log(MM)) de la colonne de chromatographie utilisée pour séparer les différentes formes de prolactine contenues dans des échantillons de sérum.

La figure 4 représente les résultats de séparations chromatographiques réalisées sur cette colonne au départ de deux sérums hyperprolactinémiques, l'un riche en macroprolactines (sérum N) et l'autre sans macroprolactine (sérum W).

La figure 5 représente les courbes de dilution réalisées selon la méthode II telle que définie plus loin dans la présente description et obtenue pour trois échantillons différents dont l'un est un sérum riche en macro-prolactine (MC2427) et les deux autres sont connus pour ne contenir que de la PRL monomérique (liquide amniotique et standard international (WHO 84/500)).

La figure 6 représente la corrélation entre les résultats de dosage de la macroprolactine obtenus selon la méthode connue de précipitation au PEG (méthode VII décrite plus loin) et ceux obtenus selon le procédé de la présente invention.

La figure 7 représente l'arbre décisionnel utilisé pour qualifier de façon discriminative un cas d'hyperprolactinémie et décider en conséquence du traitement à mettre éventuellement en oeuvre et du type de suivi à donner aux cas correspondant aux diverses hypothèses.

## Exemples

### *Matériel et méthodes*

#### *Fixation des anticorps monoclonaux 68.9G5 et 69.5.A5 sur une microplaque*

La fixation des anticorps se réalise sur des microplaques fixées à la streptavidine en suivant les étapes a, b et c décrites ci-dessous :

a) Les anticorps monoclonaux 69.5A5 et 68.9.G5 sont biotinylés par le dérivé NHS-LC-biotine (commercialisé par Pierce.Biotechnology, Inc., P.O. 117, Rockford, IL 61105, USA) suivant les recommandations fournies par le fabricant.

b) Les microplaques streptavidine sont produites en incubant une nuit dans chaque puits de la plaque (LabSystems) 200 $\mu$L d'une solution de streptavidine dissoute dans un tampon bicarbonate pH de l'ordre de 9.6. Après incubation, le contenu de chaque puits est aspiré et 200 $\mu$L d'une solution de saturation (PBS + Polyvinyl pyrrolidone 0.4%, glycine 7.5%, pH de l'ordre de 7.4) sont ensuite dispensés dans ces mêmes puits. Après une seconde incubation de 4 heures à température ordinaire, le contenu des puits est aspiré et les puits sont lavés 3 fois par un tampon PBS. Les microplaques contenant la streptavidine sont séchées sous vide, puis emballées avec un agent dessicant dans des sachets plastiques scellés à chaud et conservées à environ 4 °C.

c) La préparation des microplaques avec les anticorps biotinylés 69.5A5 ou 68.9.G5 s'effectue de la manière suivante: 200 $\mu$L d'une solution à 1 $\mu$g/ml d'anticorps biotinylé sont dispensés dans chaque puits d'une microplaque coatée par la streptavidine. Après incubation une nuit à température ordinaire, le contenu des puits est aspiré et 250 $\mu$L de solution de saturation sont dispensés (19.2 g/L acide citrique, 5 g/L d'albumine bovine, à pH de l'ordre de 5.0). Les microplaques sont incubées à température ordinaire pendant 2 heures. Le contenu des puits est aspiré et chaque puits est lavé 3 fois par 250 $\mu$L de tampon de lavage (PBS). Les microplaques lavées sont ensuite séchées sous vide, puis emballées en présence d'un agent dessicant dans des sachets en aluminium scellés à chaud. Elles sont conservées à environ 4 °C jusqu'au moment du dosage.

*Préparation du conjugué HRP*

Le conjugué est préparé par couplage de l'enzyme Horse Radish Peroxidase (HRP) à un anticorps monoclonal codé 69 2 F 11. L'enzyme activé et le protocole de conjugaison utilisé sont ceux fournis par la société Pierce.Biotechnology.

*Dosage des échantillons biologiques*

Les échantillons biologiques sont dosés suivant les méthodes I, II, III, IV, V, VI et VII décrites ci-dessous; chacune d'elle permet l'obtention d'une concentration en prolactine et/ou une estimation du contenu en macroprolactine à partir d'une courbe de calibration basée sur de la PRL monomérique (standard international) et sur l'exécution d'un algorithme de calcul simple (variable suivant la méthodologie).

Les concentrations déterminées sont exprimées en $\mu$IU de Prolactine monomérique par ml de solution (l'unité (IU) est basée sur le standard international WHO, code 84/500 (NIBSC, Potters Bar, Hertfordshire EN6 3QG, UK)).

### Methode I : Test ELISA 2 étapes avec capture par 69.5.A5 et révélation par 69.2.F11.

Le principe de la méthode de dosage (I) repose sur la technique sandwich réalisée en deux étapes.

Etape 1 : l'anticorps biotinylé immobilisé sur la microplaque (69. 5.A5) capture la PRL et/ou la MacroPRL circulante.
Etape 2 : Après lavage de la microplaque, un anticorps signal (anticorps 69.2.F11 marqué par l'enzyme HRP ) réagit avec un épitope de la PRL différent de celui reconnu par l'anticorps de capture. La lecture du test s'effectue, après séparation des compartiments libres et liés, par ajout d'un substrat (TMB de la société Kem En Tech, Dk) qui génère un signal colorimétrique directement proportionnel à la concentration mesurée. Cette lecture est réalisée, de préférence après ajout d'une solution d'arrêt, par lecture de la DO à 405 nm avec un lecteur de microplaque (Labsystems, Multiskan, P.O. Box 208, FIN-00811 Helsinki, Finland).
Protocole détaillé de la méthode de dosage : Ajouter dans différents puits de la microplaque coatée par l'anticorps 69 5 A5, selon un ordre séquentiel défini 25 $\mu$L de calibrateur à base de PRL monomérique (WHO 84/500) pour couvrir une gamme de dosage de 60 à 4000 $\mu$IU/ML environ.

a) Ajouter au puits suivant 25 $\mu$L d'échantillons biologiques à doser ;
b) Ajouter à tous les puits 100 $\mu$L de tampon PBS+0.1% gélatine ;
c) Incuber 2 heures à 25 °C ;
d) Aspirer le contenu des puits et laver 5 fois par un tampon de lavage (solution physiologique + 0.1% NP40) ;
e) Ajouter 100 $\mu$L de conjugué HRP à tous les puits ;
f) Incuber 30 minutes à 25 °C ;
g) Aspirer le contenu des puits et laver 5 fois par un tampon de lavage (solution physiologique + 0.1% NP40);
h) Ajouter 200 $\mu$L de substrat TMB;
i) Incuber 30 minutes à 25°C ;
j) Ajouter 50 $\mu$L de solution d'arrêt;
k) Agiter la microplaque et lire à 405 nm.

### Méthode II : Test ELISA 2 étapes avec capture par 68.9.G5 et révélation par 69.2.F11

Le principe de la méthode de dosage (II) est identique à celui décrit du protocole de la méthode (I). La reconnaissance de la macroprolactine capturée par l'anticorps monoclonal 68.9.G5 par le conjugué 69.2.F11 est favorisée tandis que celle de la prolactine monomérique est minimisée.

### Méthode III : Test commercial (Roche Diagnostics GmbH, D-68298 Mannheim, Germany)

Le dosage de prolactine est réalisé sur l'automate Modular Analytics, module E170 en utilisant le matériel et le protocole du fabricant.

Le principe de la méthode de dosage (III) repose sur une technique sandwich : un anticorps biotinylé capture la PRL et/ou MacroPRI circulante, tandis qu'un anticorps signal marqué par un complexe de ruthénium réagit avec un épitope de la PRL différent de celui reconnu par l'anticorps de capture. Après séparation des compartiments libres et liés, l'ajout d'un réactif spécifique génère un signal électroluminescent directement proportionnel à la concentration mesurée.

L'automate, préalablement calibré, génère automatiquement les résultats des différents dosages et les exprime en $\mu$IU/ml (WHO 84/500) de PRL monomérique.

### Méthode IV : Test ELISA 1 étape avec capture par 69.5.A5 et *révélation par 69.2.F11*

Le principe de la méthode de dosage (IV) repose sur la technique sandwich réalisée en une seule étape.

Principe : L'anticorps biotinylé immobilisé sur la microplaque (69. 5.A5) et l'anticorps signal (anticorps 69.2.F11 marqué par l'enzyme HRP ) sont mis en présence de la PRL. Cette dernière est prise en sandwich via ses épitopes spécifiques des deux anticorps. L'ordre de fixation de la PRL aux 2 anticorps est aléatoire et est principalement régi par les constantes d'association ainsi que les coefficients de diffusion relatifs à la formation de ces complexes antigène-anticorps. Après séparation des compartiments libres et liés par lavage de la microplaque, une quantification du traceur lié à la plaque est réalisée par ajout d'un substrat (TMB de la société Kem En Tech, Dk) qui génère un signal colorimétrique directement proportionnel à la quantité de traceur incorporé. La lecture de ce signal est réalisée, de préférence après ajout d'une solution d'arrêt, par lecture de la DO à 405 nm avec un lecteur de microplaque (Labsystems, Multiskan, P.O. Box 208, FIN-00811 Helsinki, Finland).

Protocole détaillé de la méthode de dosage : Ajouter dans différents puits de la microplaque coatée par l'anticorps 69 5 A5, selon un ordre séquentiel défini 25 $\mu$L de calibrateur à base de PRL monomérique (WHO 84/500) pour couvrir une gamme de dosage de 60 à 4000 $\mu$IU/ML environ.

a) Ajouter aux puits suivants 25 $\mu$L d'échantillons biologiques à doser ;
b) Ajouter à tous les puits 100 $\mu$L de tampon PBS+0.1% gélatine ;
c) Ajouter 100 $\mu$L de conjugué HRP à tous les puits;
d) Incuber 2 heures à 25 °C ou à 37°C;
e) Aspirer le contenu des puits et laver 5 fois par un tampon de lavage (solution physiologique + 0.1% NP40) ;
f) Ajouter 200 $\mu$L de substrat TMB ;
g) Incuber 30 minutes à 25°C ;
h) Ajouter 50 $\mu$L de solution d'arrêt ;
i) Agiter la microplaque et lire à 405 nm.

### Méthode V : Test ELISA 1 étape avec capture par 68.9.G5 et révélation par 69.2.F11

Le principe de la méthode de dosage (V) est identique à la méthode IV décrite ci-dessus.

### Méthode VI : Test commercial de dosage de la PRL (kit IRMA Immunotech)

### Méthode VII : Test après précipitation au PEG

Les sérums classés hyperprolactinémiques après une première analyse suivant la méthode (III) sont redosés après précipitation au PEG. Le protocole de la méthode VII utilisé est le suivant :

a) Un volume de sérum est additionné d'un volume de solution de PEG à 25% dans un tampon PBS pH 7.4.
b) Le mélange est vortexé, puis centrifugé pendant 30 minutes à 1500 g.
c) Le surnageant est prélevé et redosé en PRL sur l'automate Roche Modular.
d) Le résultat obtenu est multiplié par 2 (pour tenir compte du facteur de dilution suite au traitement par le PEG). Ce dernier représente la concentration estimée en PRL monomérique de l'échantillon. Dans le cas ou la concentration estimée est inférieure à 40% de celle trouvée en première analyse l'échantillon est supposé contenir des macroprolactines. Entre 40 et 60%, la présence de macroprolactine dans l'échantillon est incertaine.

Rendu des résultats :

Si $[PRL]_{init}$ représente la concentration en prolactine calculée en première analyse et $[PRL]_{est}$ la concentration en prolactine estimée après précipitation au PEG :

- % précipitation au PEG = 100 x (1-$[[PRL]_{est}/[PRL]_{init}$), si > 60 %, présence de macro-PRL;
- [PRL] monomérique (active)= $[PRL]_{est}$

*Exemple 1 : Dosage de la PRL monomérique dans les différents systèmes d'immunodosage*

Le but de l'exemple est d'analyser la différence de réactivité de la PRL monomérique suivant :

- l'anticorps utilisé sur la phase solide (69.5A5 ou 68.9.G5) ;
- le protocole de dosage (1 ou 2 étapes);
- la température.

On dose différentes dilutions du standard international PRL (WHO 84/500) réalisées dans un mélange de tampon PBS et de sérum de cheval (proportions : 50 % (v/v)). Les dosages ont été réalisés sur base des méthodes I et II (en utilisant deux températures d'incubation : 25 et 37°C) et sur base des méthodes IV et V (en utilisant une température de 25°C).

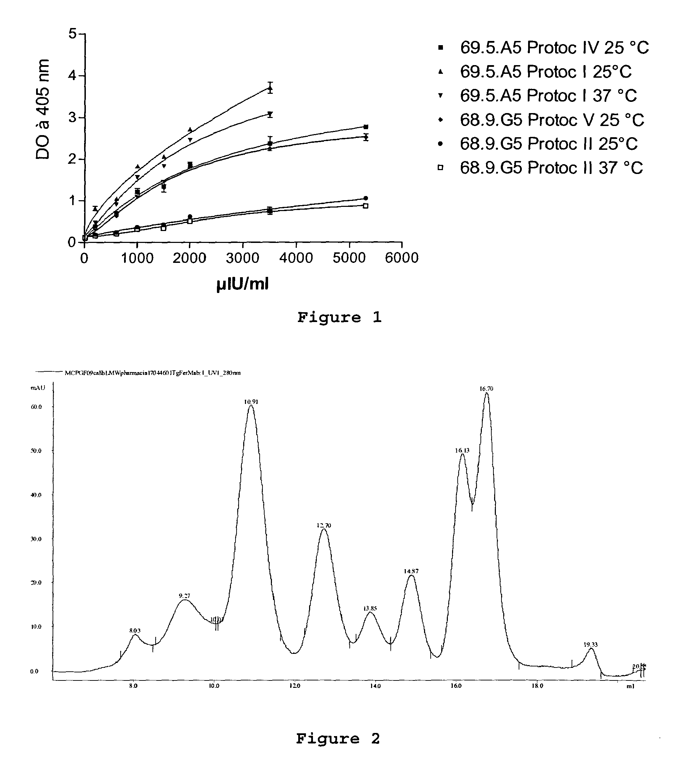

Les résultats des dosages sont présentés dans la figure 1 et montrent que la température n'affecte pas les qualités du dosage.

Les réactions immunologiques dans les systèmes d'immunodosage des méthodes I et II sont proches de l'équilibre comme l'indiquent les faibles différences observées en passant d'une température d'incubation de 25°C à 37°C.

Les courbes d'immunodosage montrent une forte différence d'immunoréactivité de la prolactine monomérique dans les systèmes d'immunodosage en 2 méthodes I et II. Par contre dans les systèmes homologues en une étape cette différence est fortement atténuée, voire inexistante. Ces observations indiquent que le processus réactionnel est important.

*Exemple 2 : Chromatographie de sérums sur tamis moléculaire et analyse immunologique des fractions chromatographiées*

Le but de l'exemple est de doser des sérums en PRL par la méthode III, de détecter les sérums classés hyperprolactinémiques et de les caractériser par la méthode VII (méthode au PEG).

On sélectionne parmi ces derniers deux sérums ; l'un riche en macroprolactines (N) et l'autre sans macroprolactines (W). Ensuite, on chromatographie sur un tamis moléculaire les deux sérums et on dose l'activité immunologique en PRL des différentes fractions avec deux paires d'anticorps présentant des caractéristiques fonctionnelles différentes (paire 69.5.A5-69.2.F11 (protocole I) et paire 68.9.G5-69.2.F11 (méthode II)).

Enfin, on met en évidence la différence de réactivité entre les différentes formes chromatographiées dans les deux systèmes d'immunodosage.

Les différentes formes de prolactine circulantes présentes dans un échantillon de sérum peuvent être séparées en fonction de leur taille par chromatographie sur un tamis moléculaire. Le profil d'élution est établi par immunodosage des différentes fractions chromatographiques.

*Détermination des performances de la colonne*

La chromatographie est réalisée sur une colonne superdex 200 (HR 10/30 de Pharmacia) d'une contenance de 20 ml équilibrée avec un tampon PBS. La colonne est préalablement caractérisée par détermination des volumes d'élution d'un set de calibrateurs protéiques (Calibrage Pharmacia LMW ref 17044601) et de quelques références internes, Tg, ferritine et une IgG. Le profil d'élution est représenté à la figure 2.

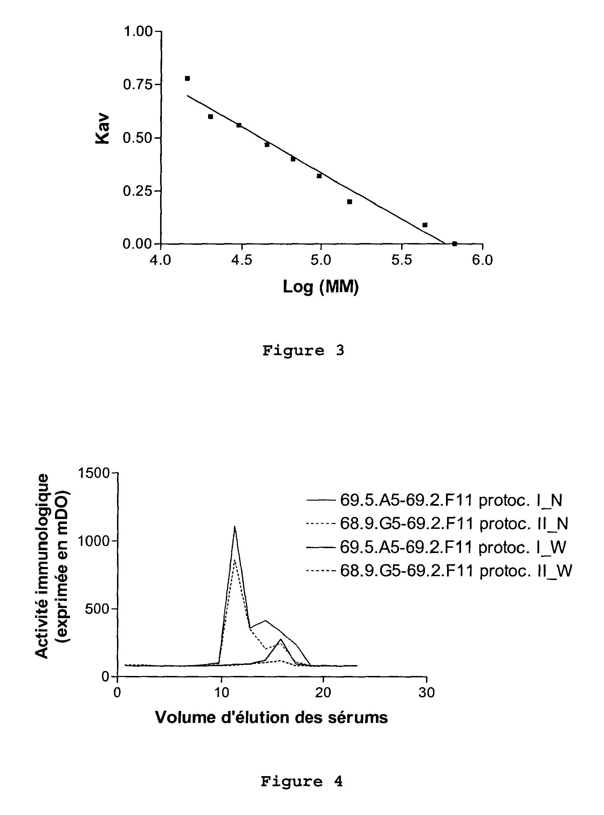

Les performances chromatographiques de la colonne (Kav vs Log (MM)) sont déterminées à partir de la table 1 ci-dessous et du graphique présenté en figure 3.

Table 1

| Substance | Masse moléculaire | Volume d'élution (ml) | Kav : (Ve-V0) / (Vt-V0) |
|---|---|---|---|
| Thyrogobuline (Tg) | 669000 | 8.03 | 0.080 |
| Ferritine | 440000 | 9.27 | 0.170 |
| IgG | 150000 | 10.91 | 0.300 |
| Phosphorylase B | 97000 | 12.70 | 0.440 |
| Albumine | 66000 | 13.85 | 0.530 |
| Ovalbumine | 45000 | 14.87 | 0.610 |
| Carbonic anhydrase | 30000 | 16.13 | 0.700 |

Suite de tableau

| Substance | Masse moléculaire | Volume d'élution (ml) | Kav : (Ve-V0) / (Vt-V0) |
|---|---|---|---|
| Trypsine inhibiteur | 20100 | 16.70 | 0.750 |
| Alpha-lactalbumine | 14400 | 19.33 | 0.950 |

*Chromatographie des sérums*

500 µl de sérum sont injectés sur la colonne à un débit de 0.4 ml/min. L'élution est réalisée en conservant ce débit ainsi que le tampon d'équilibrage. Des fractions chromatographiques de 500 µl sont collectées directement à la sortie de la colonne. Le profil d'élution est obtenu en déterminant l'activité immunologique des différentes fractions en suivant les méthodes d'immunodosages I et II.

Trois pics ont été mis en évidence lors de l'établissement des profils d'élution. Les pics ont été caractérisés au niveau chromatographique sur base de la relation entre le Kav et la masse moléculaire (voir table 2 ci-dessous et figure 4).

Table 2

| | Volume d'élution | Kav | MM estimée | Identification |
|---|---|---|---|---|
| Pic 1 | 11.25 ml | 0.22 | - 180000 | Macro PRL (IgG + PRL) |
| Pic 2 | 14.25 ml | 0.43 | - 60000 | PRL-PRL |
| Pic 3 | 15.70 ml | 0.53 | - 35000 | PRL |

Ces résultats obtenus sur le sérum négatif en macro-prolactine (W) confirment les résultats obtenus dans la 1ère série de manipulation, à savoir une plus grande réactivité immunologique de la prolactine monomérique dans le système avec 69.5.A5 (protocole 1) que dans le système avec 68.9.G5.

D'un autre côté, contrairement à la prolactine monomérique, la macro-prolactine exprime une réactivité immunologique semblable dans ces 2 systèmes.

*Exemple 3 : Caractérisation de différentes formes circulantes de PRL par un test de dilution*

Le but de l'exemple est d'identifier différentes formes circulantes de PRL à travers un test en dilution réalisé sur l'échantillon à caractériser.

On sélectionne trois échantillons de nature différente contenant des formes circulantes de PRL susceptibles de présenter des immunoréactivités variables. L'un est un sérum riche en macro-prolactine (MC2427) et les deux autres sont connus pour ne contenir que de la PRL monomérique. Ces derniers, liquide amniotique et standard international (WHO 84/500), peuvent toutefois présenter des taux de glycosylation ou de phosphorylation variables.

On réalise ensuite une série de dilution de 2 en 2 de chaque échantillon et on les dose suivant la méthode I.

Pour chaque échantillon, on porte en graphique la DO obtenue en fin de dosage en fonction de la concentration estimée sur base de la concentration initiale de l'échantillon (calculée suivant la méthode III ou IV ou suivant la valeur de référence fournie (cas du standard WHO)) et sur base du facteur de dilution.

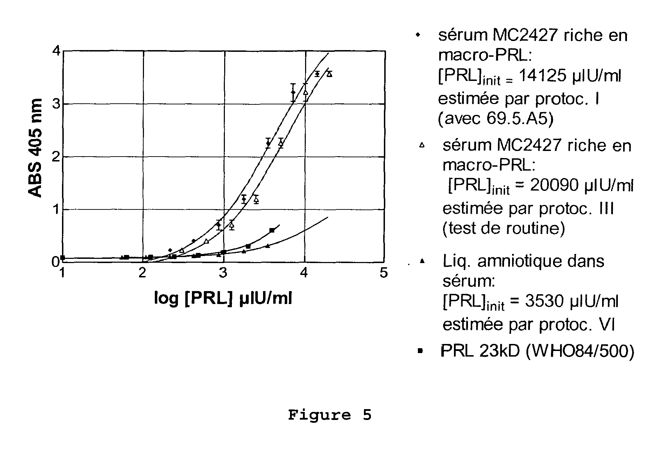

Les courbes de dilution représentées à la figure 5 montrent des variations de réactivité immunologique pour les trois types d'échantillon testés. Ces variations se reflètent à travers la pente obtenue dans les courbes de dilution.

Par ces données, il est possible d'identifier différentes formes circulantes de prolactine dans un échantillon en réalisant une courbe de dilution de ce dernier suivant la méthode II, et la présence de macroprolactine est clairement attestée par une immunoréactivité nettement plus importante que celle déterminée par le test de routine.

*Exemple 4 : Corrélation entre l'estimation du contenu en macro-PRL calculé par la méthode au PEG et le contenu en macro-PRL évalué par un système d'immunodosage (protocole II avec 68.9.G5)*

Le but de l'exemple est d'établir une méthode d'identification de macro-PRL basée sur le procédé d'immunodosage de l'invention (une méthode de routine du dosage de PRL (méthodes I ou III) + le dosage avec 68.9.G5 (protocole II)) et de corréler cette dernière avec la méthode au PEG pour valider notre système.

40 sérums ont été dosés et caractérisés sur un automate Modular ™ en utilisant la méthode III et la méthode VII. Cette population de sérums a ensuite été dosée en suivant les méthodes I et II. Des concentrations en prolactine ont été calculées sur base de ces deux protocoles. L'analyse doit être réalisée de la manière suivante :

1. si concentration estimée via la méthode III ou I < seuil d'hyperprolactinémie → échantillon normal ;

2. si concentration estimée via la méthode III ou I > seuil d'hyperprolactinémie → suspicion d'une hyperprolactinémie (PRL monomérique trop élevée → utiliser résultat du dosage via la méthode II,

    a. si concentration via la méthode II similaire à celle obtenue avec la méthode I ou III → hyperprolactinémie sans présence de macroprolactine ;

    b. si concentration via la méthode II significativement supérieure à celle obtenue via la méthode I ou III → présence de macroprolactine (plus cette différence est importante et plus la concentration en macroprolactine de l'échantillon est élevée) .

Le classement des échantillons est présenté selon un code couleur exprimant leur contenu en macro-prolactine. Cette codification est décrite dans le tableau 1. En effet, les critères reconnus pour la méthode au PEG (positif > 60%, négatif < 40 %) seront corrélés au rapport enregistré par le procédé de l'invention :

$$\left[\ [PRL]_{protoc.\ II} - [PRL]_{protoc.\ I}\ \right] / [PRL]_{protoc.\ II}$$

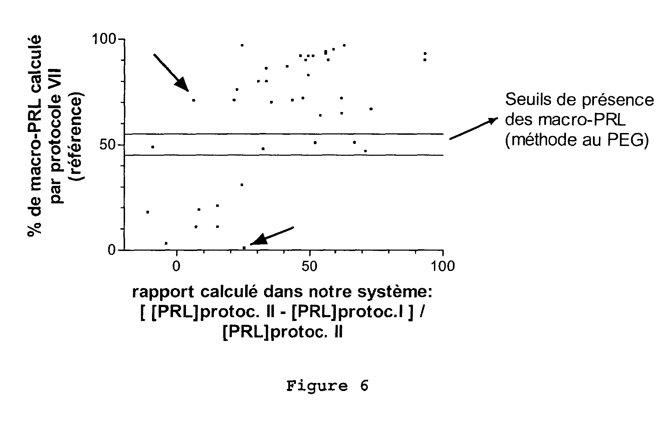

Les résultats obtenus sont présentés dans la table 3 ci-dessous et à la figure 6.

Les critères de détection de la méthode au PEG sont conservés : présence de macroprolactine si % de précipitation supérieur à 60%, absence si < 40%, douteux entre 40% et 60%.

A l'examen des valeurs du rapport donné par la méthode de l'invention, le critère provisoire de présence de macroprolactine est de > 20. Selon ces critères, la corrélation entre les résultats obtenus par les deux méthodes amène aux conclusions suivantes :

- sur 27 positifs selon les critères PEG, 26 sont positifs selon le procédé de l'invention ;
- sur 9 négatifs selon les critères PEG, 7 le sont également selon le procédé de l'invention ;
- les 4 échantillons douteux au PEG sont classés positifs selon le procédé de l'invention ;
- Deux échantillons fléchés (voir graphe ci-dessous) donnent des résultats opposés, un premier étant négatif par la méthode PEG et positif par le procédé de l'invention et l'inverse pour le second : ceci pourrait laisser supposer la présence d'isoformes de PRL rares telles entre autres que des dimères ou des fragments.

Il existe une bonne corrélation entre le classement des échantillons via les méthodes au PEG (méthode VII) et ceux calculés via le procédé de l'invention.

Table 3

| Echantillon | Référence: PEG | Rapport calculé par notre méthode x 100 | Echantillon | Référence: PEG | Rapport calculé par notre méthode x 100 |
|---|---|---|---|---|---|
| Sérum 1 | 87.3 | 41 | Sérum 24 | 90.1 | 48 |
| Sérum 2 | 90.3 | 93 | Sérum 25 | 11.1 | 7 |
| Sérum 3 | 90.1 | 57 | Sérum 26 | 20.5 | 15 |
| Sérum 4 | 94.3 | 56 | Sérum 27 | 18.9 | 8 |
| Sérum 5 | 71.0 | 6 | Sérum 28 | 0.5 | 25 |
| Sérum 6 | 85.9 | 33 | Sérum 29 | 18.5 | -11 |
| Sérum 7 | 79.9 | 30 | Sérum 30 | 48.7 | -9 |
| Sérum 8 | 70.8 | 43 | Sérum 31 | 76.0 | 22 |
| Sérum 9 | 70.6 | 21 | Sérum 32 | 51.0 | 52 |
| Sérum 10 | 80.0 | 33 | Sérum 33 | 71.8 | 62 |
| Sérum 11 | 97.3 | 63 | Sérum 34 | 92.8 | 93 |
| Sérum 12 | 92.7 | 56 | Sérum 35 | 70.4 | 35 |
| Sérum 13 | 92.2 | 49 | Sérum 36 | 97.4 | 24 |
| Sérum 14 | 92.0 | 51 | Sérum 37 | 92.0 | 46 |
| Sérum 15 | 31.0 | 24 | Sérum 38 | 94.9 | 59 |
| Sérum 16 | 3.0 | -4 | Sérum 39 | 83.4 | 49 |
| Sérum 17 | 11.2 | 15 | Sérum 40 | 72.5 | 47 |
| Sérum 18 | 65.0 | 62 | Légende : Code couleur | | |
| Sérum 19 | 63.6 | 54 | Echantillon positif | | |
| Sérum 20 | 67.2 | 73 | Echantillon négatif | | |
| Sérum 21 | 48.2 | 32 | Echantillon douteux | | |
| Sérum 22 | 51.1 | 67 | | | |
| Sérum 23 | 47.0 | 71 | | | |

**Exemple 5 :** *Recherche de spécificité des immunodosages de la prolactine et arbre décisionnel*

Les producteurs d'immunodosages de PRL cherchent à distribuer des méthodes où l'interférence de la macro-prolactine est minimale sans pouvoir toutefois l'éliminer. Dès lors, la mise en évidence d'une hyperprolactinémie fait systématiquement se poser l'hypothèse d'une telle interférence.

C'est donc d'emblée que le recours à une méthode d'identification de la macroprolactine se pose tant aux cliniciens qu'aux laboratoires de biologie clinique.

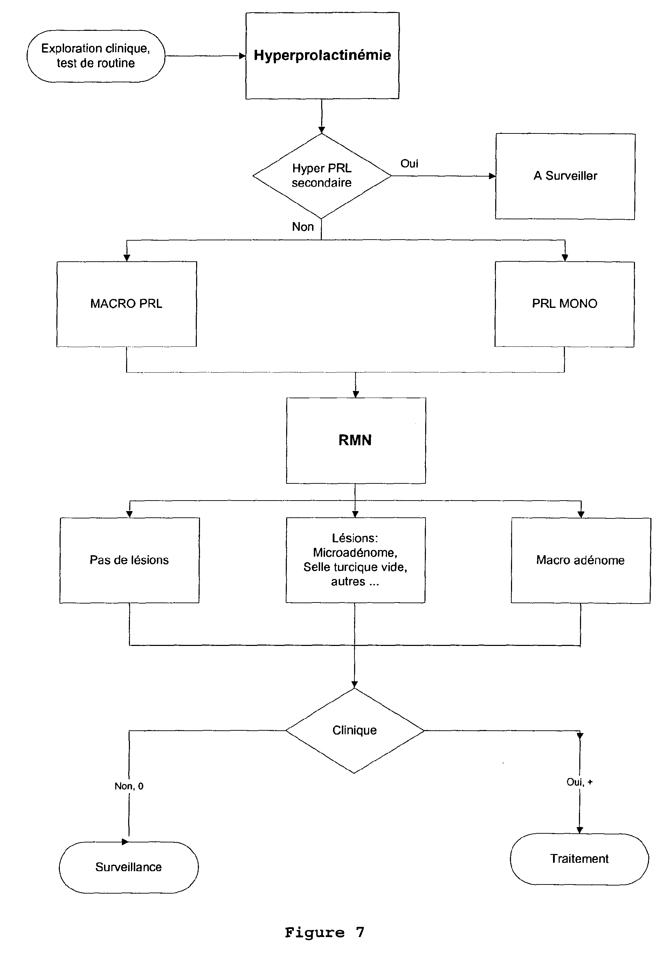

L'arbre décisionnel illustré à la figure 7 décrit cette nécessité pour la bonne conduite des explorations complémentaires et des économies à faire (imagerie médicale, tests de biologie endocrinienne et métabolique et surtout traitement) dès lors qu'une situation de macroprolactinémie bénigne est clairement établie par le procédé de l'invention qui peut être combiné aux étapes proposées dans cet arbre décisionnel et effectuées par le praticien.

## Revendications

1. Procédé de détection et/ou de dosage de macroprolactines et de prolactine monomérique dans un échantillon biologique extrait d'un patient humain ou animal qui comprend les étapes suivantes :

   a) immunodosage par la capture préférentielle de la prolactine sous une forme monomérique présente dans l'échantillon biologique à doser par des anticorps ou des portions hypervariables d'anticorps reconnaissant spécifiquement la prolactine monomérique et évaluation d'un premier titre (titre 1) en prolactine dans l'échantillon biologique par interpolation du signal obtenu dans le dosage de l'échantillon biologique avec ceux obtenus lors d'un dosage d'une courbe standard ;
   b) immunodosage par la capture préférentielle de la prolactine sous forme de macroprolactine présente dans l'échantillon biologique à doser par des anticorps reconnaissant préférentiellement la macroprolactine et évaluation d'un second titre (titre 2) en prolactine dans l'échantillon biologique par interpolation du signal obtenu dans le dosage de l'échantillon biologique avec ceux obtenus lors du dosage d'une courbe standard; et ;
   c) établissement d'une corrélation des immunodosages obtenus à la présence ou l'absence d'une hyperprolactinémie chez le patient dans le cas où :

      - si le titre 1 est inférieur à la limite supérieure de l'intervalle de normalité, on considère l'échantillon biologique comme obtenu d'un patient sain;
      - si le titre 1 est supérieur à la limite supérieure de l'intervalle de normalité, on considère que l'échantillon biologique est obtenu d'un patient souffrant d'une hyperprolactinémie et/ou d'une macroprolactinémie ;
      - si le titre 2 est comparable au titre 1, on considère que l'échantillon biologique est obtenu d'un patient souffrant d'une hyperprolactinémie;
      - si le titre 2 est significativement supérieur au titre 1, on considère que l'échantillon biologique comporte des macroprolactines induisant une macroprolactinémie d'autant plus élevée que le différentiel du titre 2 au titre 1 est important et si les titres 1 et 2 sont très supérieurs aux limites supérieures des intervalles de normalité et que la différence entre le titre 2 et le titre 1 révèle une macroprolactinémie incompatible avec une prolactinémie du titre 1, on considère que l'échantillon biologique comporte des macroprolactines et est obtenu d'un patient souffrant d'une hyperprolactinémie, éventuellement associée à la présence d'une macroprolactinémie.

2. Procédé selon la revendication 1,
   **caractérisé en ce que** les anticorps sont des anticorps monoclonaux.

3. Procédé selon la revendication 2,
   **caractérisé en ce que** les anticorps monoclonaux reconnaissant préférentiellement les macroprolactines de l'immunodosage de l'étape b sont des anticorps produits par l'hybridome identifié par le numéro de dépôt LMBP 6348CB).

4. Procédé selon l'une quelconque des revendications précédentes, **caractérisé en ce que** l'immudosage de l'étape a et/ou l'étape b du procédé comporte une étape de capture des macroprolactines et/ou de la prolactine monomérique par les anticorps ou les portions hypervariables des anticorps, suivie d'une étape de lavage, de manière à éliminer tous les composants susceptibles d'interférer avec le dosage préférentiel des macroprolactines et/ou de la prolactine monomérique.

5. Procédé selon l'une quelconque des revendications précédentes, **caractérisé en ce que** l'échantillon biologique est choisi parmi le groupe constitué par le sang veineux ou capillaire, le sérum ou le plasma.

6. Procédé selon l'une quelconque des revendications précédentes, **caractérisé en ce que** les anticorps ou les portions hypervariables des anticorps sont fixés directement ou indirectement sur un support solide choisi parmi le groupe constitué par des plaques multipuits, des billes ou particules, de préférence magnétiques ou des tubes de préférence en plastique.

**7.** Procédé de détection et/ou de dosage de macroprolactines dans un échantillon biologique extrait d'un patient qui comporte une immuno-détection et/ou un immunodosage par la capture préférentielle de la prolactine sous forme de macroprolactines, éventuellement présentes dans l'échantillon biologique à détecter et/ou à doser par des anticorps reconnaissant préférentiellement ladite prolactine sous forme de macroprolactines et éventuellement évaluation d'un titre en macroprolactines dans l'échantillon biologique par interpolation du signal obtenu dans le dosage de l'échantillon biologique avec ceux obtenus lors d'un dosage d'une courbe standard

**8.** Procédé de discrimination des macroprolactines par rapport à la prolactine sous forme monomérique dans un échantillon biologique, **caractérisé en ce qu'**il comprend les étapes suivantes :

- on détermine une première concentration en prolactine $[PRL]_I$ selon un immunodosage commercial permettant une capture préférentielle de la prolactine monomérique par rapport à des macroprolactines;
- on détermine une deuxième concentration en prolactine $[PRL]_{II}$ selon le procédé de l'une quelconque des revendications précédentes, 1 à 6, ou une détection des macroprolactines selon le procédé de la revendication 7 et on détermine, de préférence par un algorithme adapté un rapport R= $[PRL]_{II}$ - $[PRL]_I$ /$[PRL]_{II}$ et on établit un seuil de positivité du facteur R pour déterminer la présence éventuelle de macroprolactines dans l'échantillon biologique par lequel si R = 0, l'échantillon biologique ne contient que de la prolactine sous forme monomérique

**9.** Anticorps monoclonal ou portion hypervariable de cet anticorps monoclonal, reconnaissant préférentiellement les macroprolactines et obtenu par l'hybridome correspondant au n° de dépôt LMBP 6348CB.

**10.** Trousse (kit of parts) comprenant l'anticorps selon la revendication 9 et un anticorps ou une portion hypervariable d'anticorps reconnaissant spécifiquement la prolactine monomérique.

**11.** Trousse de détection et/ou de dosage de prolactine monomérique et/ou de macroprolactines comportant les éléments de la trousse de la revendication 10 ou l'anticorps de la revendication 9 et éventuellement une phase solide fixant éventuellement lesdits anticorps et des moyens pour la mise en oeuvre du procédé de détection et/ou de dosage selon l'une quelconque des revendications présentes 1 à 8, en particulier des solutions standards contenant des concentrations variables en prolactine monomérique dans une matrice adaptée aux échantillons à doser et éventuellement des échantillons de contrôle.

**12.** Appareillage de préférence un automate, de détection et/ou de dosage de prolactine monomérique et/ou de macroprolactines comportant l'anticorps selon la revendication 9 ou la trousse selon les revendication 10 ou 11 et éventuellement une phase solide fixant éventuellement lesdits anticorps et des moyens pour la mise en oeuvre du procédé de détection et/ou du dosage selon l'une quelconque des revendications précédentes 1 à 8, en particulier des solutions standards contenant des concentrations variables en prolactine monomérique dans une matrice adaptée aux échantillons à doser et éventuellement des échantillons de contrôle.

**13.** Hybridome produisant l'anticorps selon la revendication 9 correspondant au n° de dépôt LMBP 6348CB.

Figure 1

Figure 2

Figure 3

Figure 4

**Figure 5**

- sérum MC2427 riche en macro-PRL:
  $[PRL]_{init}$ = 14125 µlU/ml
  estimée par protoc. I
  (avec 69.5.A5)

- sérum MC2427 riche en macro-PRL:
  $[PRL]_{init}$ = 20090 µlU/ml
  estimée par protoc. III
  (test de routine)

- Liq. amniotique dans sérum:
  $[PRL]_{init}$ = 3530 µlU/ml
  estimée par protoc. VI

- PRL 23kD (WHO84/500)

Figure 6

Exploration clinique, test de routine → **Hyperprolactinémie**

Hyper PRL secondaire — Oui → A Surveiller

Non

MACRO PRL                    PRL MONO

**RMN**

Pas de lésions          Lésions: Microadénome, Selle turcique vide, autres ...          Macro adénome

Clinique

Non, 0                                                    Oui, +

Surveillance                                            Traitement

Figure 7

**Office européen des brevets**

## RAPPORT DE RECHERCHE EUROPEENNE

Numéro de la demande

EP 04 44 7260

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | JP 2000 111551 A (DAINICHISEIKA CHEM COLOR) 21 avril 2000 (2000-04-21) * abrégé * ----- | 1-13 | G01N33/74 G01N33/53 C07K16/26 |
| A | US 6 083 753 A (INSERN; APPLIED RESEARCH SYSTEMS ARS) 4 juillet 2000 (2000-07-04) * colonne 1 - colonne 13 * ----- | 1-13 | |
| A | MOUNIER, C. ET AL: "Macroprolactinemia associated with prolactin adenoma" HUMAN REPRODUCTION, vol. 18, no. 4, 2003, pages 853-857, XP002349154 * page 853 - page 854 * ----- | 1-13 | |
| A | DE SCHEPPER, J. ET AL: "Clinical and biological characterization of macroprolactinemia with and without prolactin-IgG complexes" EUROPEAN JOURNAL OF ENDOCRINOLOGY, vol. 149, 2003, pages 201-207, XP002349155 * le document en entier * ----- | 1-13 | |

**DOMAINES TECHNIQUES RECHERCHES (Int.Cl.7)**

G01N

Le présent rapport a été établi pour toutes les revendications

| Lieu de la recherche | Date d'achèvement de la recherche | Examinateur |
|---|---|---|
| Munich | 13 octobre 2005 | Moreno de Vega, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**EP 1 659 406 A1**

**ANNEXE AU RAPPORT DE RECHERCHE EUROPEENNE
RELATIF A LA DEMANDE DE BREVET EUROPEEN NO.**

EP 04 44 7260

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

13-10-2005

| Document brevet cité au rapport de recherche | Date de publication | Membre(s) de la famille de brevet(s) | Date de publication |
|---|---|---|---|
| JP 2000111551 A | 21-04-2000 | AUCUN | |
| US 6083753 A | 04-07-2000 | US 6083714 A | 04-07-2000 |

EPO FORM P0460

Pour tout renseignement concernant cette annexe : voir Journal Officiel de l'Office européen des brevets, No.12/82